# EUROPEAN PATENT APPLICATION

(11) **EP 3 991 925 A1**
(43) Date of publication of application: **04.05.2022**
(21) Application number: 20205372.4
(22) Date of filing: 03.11.2020
(51) Int. Cl.: B25J 15/00, B25J 11/00, G01N 1/02, G01N 1/10, G01N 35/00, C12M 1/00, C12M 1/30, G01N 1/20, G01N 1/22, B67C 3/22

(54) **MANIPULATING DEVICE FOR A ROBOT, ROBOT HAVING A MANIPULATING DEVICE FOR A RECEPTACLE TREATMENT MACHINE, RECEPTACLE TREATMENT MACHINE HAVING A ROBOT AND QUALITY CONTROL METHOD**

(71) Applicant: Sidel Participations, 76930 Octeville-sur-Mer (FR)
(72) Inventor: BORGESE, Rossana, 43126 Parma (IT); TASORA, Alessandro, 43126 Parma (IT); FUSAI, Dario, 43126 Parma (IT); PARMA, Filippo, 43126 Parma (IT); CENCI, Mattia, 43126 Parma (IT)
(74) Representative: Sidel Group

(57) **Abstract**

There is described a manipulating device (34) for a robot (19) for operation within a receptacle treatment machine (1), even more particular for operation within an aseptic and/or sterile environment of a receptacle treatment machine. The manipulating device (34) comprises a robotic arm (35) and an end effector unit (36) mounted to the robotic arm (35). The end effector unit (36) is controllable into at least a first operating condition within which the end effector unit (36) is configured to manipulate a swab device (20) and/or a second operating condition within which the end effector unit (36) is configured to manipulate an fluid filtering and/or sampling device (21) and/or a third operating condition within which the end effector unit (36) is configured to manipulate a sampling dish (22).

## Description

### TECHNICAL FIELD

The present invention relates to a manipulating device for a robot for a receptacle treatment machine, in particular for operation within the receptacle treatment machine, even more particular for operation within a sterile and/or aseptic environment of the receptacle treatment machine.

Advantageously, the present invention further relates to a robot for a receptacle treatment machine, in particular for operation within the receptacle treatment machine, even more particular for operation within a sterile and/or aseptic environment of the receptacle treatment machine.

Advantageously, the present invention also relates to a receptacle treatment machine, in particular a receptacle treatment machine for the filling and capping of receptacles, having a robot.

Advantageously, the present invention is also directed to a quality control method.

### BACKGROUND ART

Pourable food products are known to be packaged into receptacles such as bottles within automatic receptacle treatment machines.

Such receptacle treatment machines comprise at least an isolation chamber separating an inner environment from an outer environment, a filling apparatus arranged within the inner environment for filling the receptacles with the pourable food product and a capping apparatus for applying closures onto the filled receptacles.

Prior to the operation of the receptacle treatment machine the inner environment is cleaned or sterilized so as to guarantee that the pourable food product is packaged within the inner environment under a controlled condition.

For reasons of quality control at the end of a production cycle, the cleanliness of the inner environment is controlled by swapping at least one swapping device over a critical surface within the inner environment and/or by exposing a sampling dish containing a culture media and/or by taking samples of a fluid used within the inner environment. Then, the taken samples are analyzed so as to determine the cleanliness within the inner environment at the end of the processing in order to understand on whether the cleanliness was maintained according to the required standards or on whether problems may have arisen during operation of the receptacle treatment machine.

An inconvenience is found in that the test results are only available at the end of a production cycle, which, may also last several days.

A further inconvenience is considered to rely on the fact that if quality issues may have arisen, it is difficult or even impossible to deduce on when the quality issues started to evolve and to compromise the production process.

Thus, a desire is felt in the sector to provide means which may allow to control the cleanliness within the inner environment continuously throughout the production process.

Alternatively or in addition, a desire is felt in the sector to also allow an assessment of the samples taken during the production process.

### DISCLOSURE OF INVENTION

It is therefore an object of the present invention to provide means to overcome, in a straightforward and low-cost manner, the aforementioned drawbacks.

In particular, it is therefore an object of the present invention to provide means so as to allow a continuous monitoring of the cleanliness by taking samples within the inner environment during the production process and/or to also analyze the taken samples during the production process.

Advantageously, it is therefore an object of the present invention to provide a receptacle treatment machine to overcome, in a straightforward and low-cost manner, the aforementioned drawbacks.

According to the present invention, there is provided a manipulating device as claimed in claim 1.

Preferred non-limiting embodiments are claimed in the claims being directly or indirectly dependent on claim 1.

According to the present invention, there is also provided a robot according to any one of claims 10 to 12.

According to the present invention, there is provided a receptacle treatment machine according to any of claims 13 and 14.

According to the present invention, there is also provided a quality control method according to claim 15.

### BRIEF DESCRIPTION OF THE DRAWINGS

A non-limiting embodiment of the present invention will be described by way of example with reference to the accompanying drawings, in which:
Figure 1 is a schematic top view of a receptacle treatment machine having a manipulating device according to the present invention, with parts removed for clarity;
Figure 2 is a perspective view of portions of the receptacle treatment machine of Figure 1, with parts removed for clarity;
Figure 3 is a perspective view of the manipulating device according to the present invention, with parts removed for clarity;
Figure 4 is a perspective view of a further detail of the manipulating device according to the present invention, with parts removed for clarity;
Figure 5 is a perspective view of an even other detail of the manipulating device according to the present invention, with parts removed for clarity;
Figures 6a to 6c are perspective views of a detail of the manipulating device according to the present invention in different operating conditions, with parts removed for clarity;
Figures 7 and 8 are respectively a front and bottom view of some portions of the manipulating device according to the present invention and when being in a determined operating condition, with parts removed for clarity; and
Figure 9 is a perspective view of a further detail of the manipulating device according to the present invention, with parts removed for clarity.

### BEST MODES FOR CARRYING OUT THE INVENTION

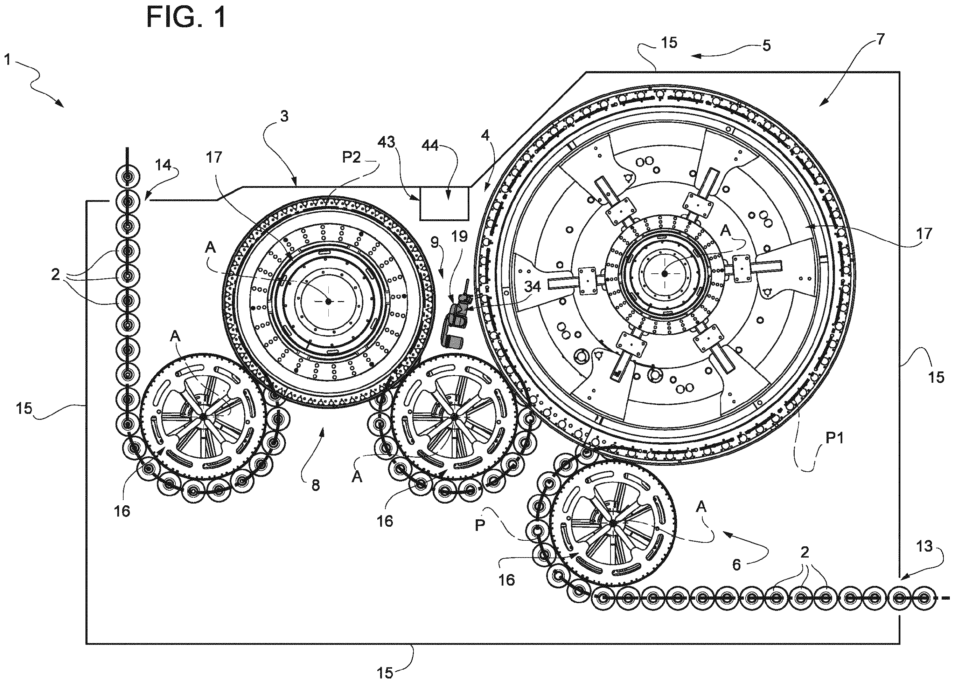

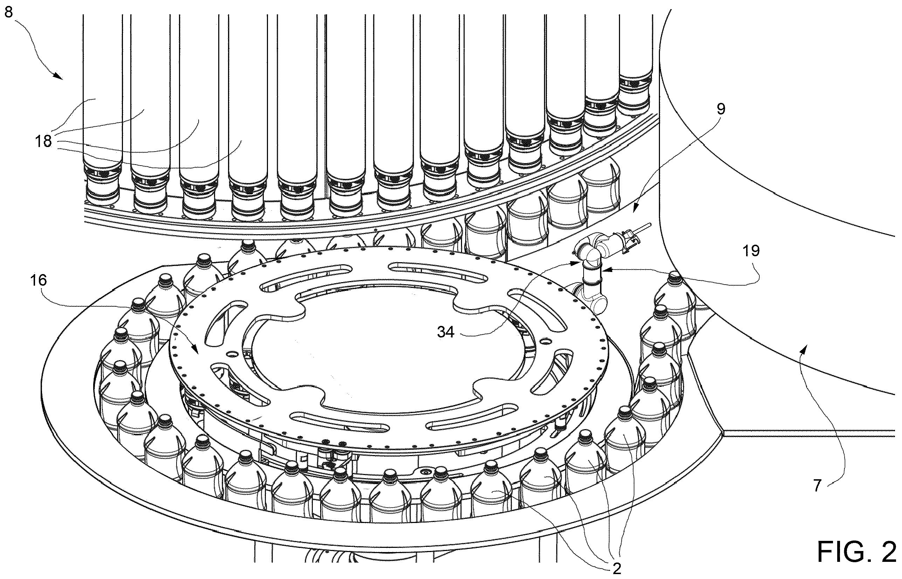

Number 1 in Figure 1 indicates as a whole a receptacle treatment machine, in particular an automatic receptacle treatment machine, for the treatment of receptacles 2 (see Figure 2), such as bottles, jars, vessels, containers or the like, in particular being made of base components, like glass, paper or cardboard, plastics, aluminum, steel, and composites.

In more detail, receptacle treatment machine 1 may be configured to:
- fill receptacles 2 with a pourable food product, such as carbonated liquids (e.g. sparkling water, soft drinks and beer), non-carbonated liquids (including still water, juices, teas, sport drinks, wine, milk, etc.), emulsions and beverages containing pulps; and/or
- to apply closures, such as crown corks, screw caps, sports caps, stoppers or similar (and being produced from a variety of materials such as plastics and metal).

According to some preferred non-limiting embodiments, receptacle treatment machine 1 may be configured to fill and/or apply closures onto receptacles 2 within a sterile and/or aseptic environment.

With particular reference to Figures 1 and 2, receptacle treatment machine 1 comprises at least:
- an isolation chamber 3 separating an inner (sterile and/or aseptic) environment 4 from an outer environment 5;
- a conveying apparatus 6 configured to advance (a succession of) receptacles 2 along an advancement path P within inner environment 4 and/or isolation chamber 3;
- a filling apparatus 7 at least partially arranged within inner environment 4 and configured to fill receptacles 2 with the pourable product during their advancement along at least a filling portion P1 of advancement path P; and/or
- a capping apparatus 8 at least partially arranged within inner environment 4 and configured to apply closures onto receptacles 2 during their advancement along at least a capping portion P2 of advancement path P, in particular capping portion P2 being arranged downstream of filling portion P1 along advancement path P.

Furthermore, receptacle treatment machine 1 may comprise a conditioning unit configured to control an ambient condition, such as temperature and/or humidity and/or sterility and/or cleanliness and/or an airflow (direction), within inner environment 4.

Advantageously, receptacle treatment machine 1 also comprises a quality control apparatus 9 configured to execute operations allowing to determine the cleanliness (sterility) within inner environment 4, in particular during operation of receptacle treatment machine 1 (i.e. during the treatment of receptacles 2).

In more detail, isolation chamber 3 comprises an inlet 13 and an outlet 14 designed to allow for respectively feeding and discharging receptacles 2 to and from inner environment 4.

Furthermore, isolation chamber 3 may comprise a plurality of walls 15 (only some shown) delimiting inner environment 4, in particular delimiting inner environment 4 from six faces, and carrying inlet 13 and outlet 14.

Preferentially, one or more walls 15 define a base (bottom portion) of receptacle treatment machine 1, in particular of isolation chamber 3.

In more detail, conveying apparatus 6 may be configured to advance receptacles 2 from inlet 13 to outlet 14, and in particular is designed to receive receptacles 2 at inlet 13 and to discharge receptacles 2 at outlet 14.

In further detail, conveying apparatus 6 may comprise one or more star wheels 16, in the example shown three, and/or one or more conveying carousels 17, in the example shown two, each star wheel 16 and each conveying carousel 17 being rotatable around a respective rotation axis A, in particular having a vertical orientation, and each one being configured to advance receptacles 2 along a respective (arc-shaped) portion of advancement path P.

Preferentially, one conveying carousel 17 is associated to filling apparatus 7 and defines (at least partially) filling portion P1 and/or one conveying carousel 17 is associated to capping apparatus 8 and defines (at least partially) capping portion P2.

Even more preferentially, at least one star wheel 16 is arranged upstream of each conveying carousel 17 and at least one star wheel 16 is arranged downstream of one respective conveying carousel 17 along advancement path P.

In more detail, filling apparatus 7 may comprise a plurality of filling units (not shown and known as such) arranged within inner environment 4 and configured to fill receptacles 2 with the pourable product during advancement of receptacles 2 along filling portion P1.

In particular, the filling units are arranged on the respective conveying carousel 17, in particular the filling units being equally spaced about the respective rotation axis A.

Preferentially, each filling unit comprises a retaining element, such as a gripping element or a pedestal, designed to retain one respective receptacle 2 during advancement along the filling portion P1 and a filling valve designed to direct the pourable product into the respective receptacle 2 during its advancement along filling portion P1.

In further detail, each capping apparatus 8 may comprise a plurality of capping units (known as such) configured retain receptacles 2 and to apply (and fasten) the closures on receptacles 2 during their advancement along capping portion P2.

Preferentially, the capping units are arranged on the respective conveying carousel 17, and in particular are equally spaced about the respective rotation axis A.

In even further detail, each capping unit may comprise a respective retaining element, such as a gripping element or a pedestal, configured to retain one respective receptacle 2 during advancement along the capping portion P2 and a respective capping head 18 configured to apply and to fasten one respective closure on the respective receptacle 2.

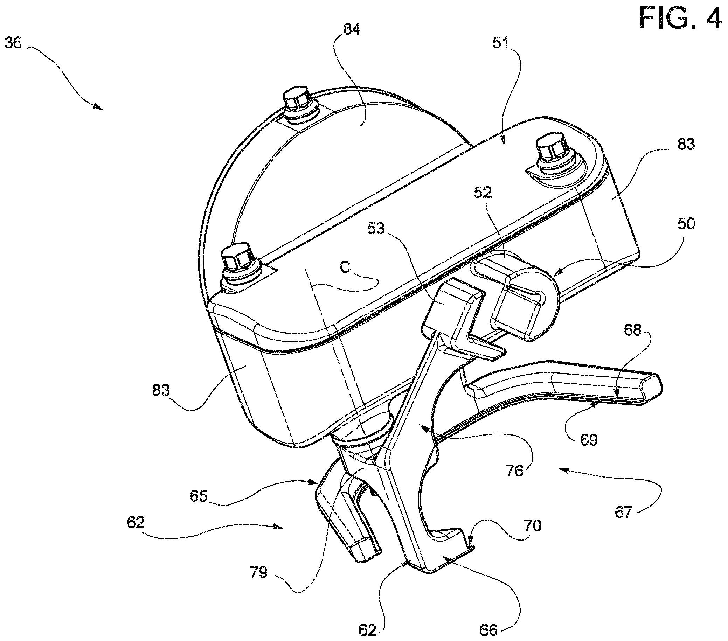

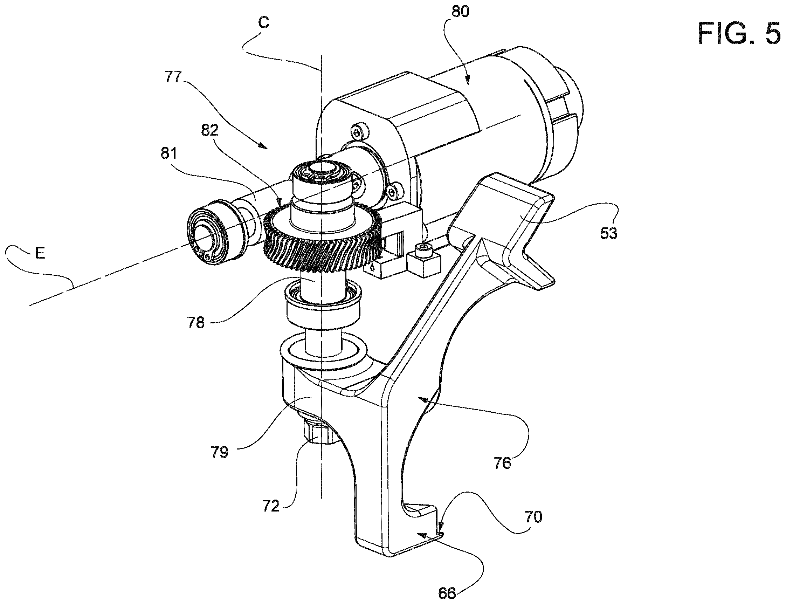

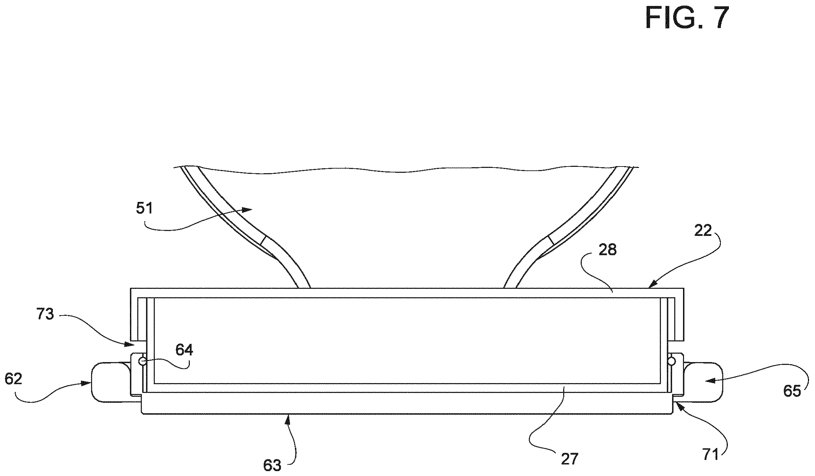

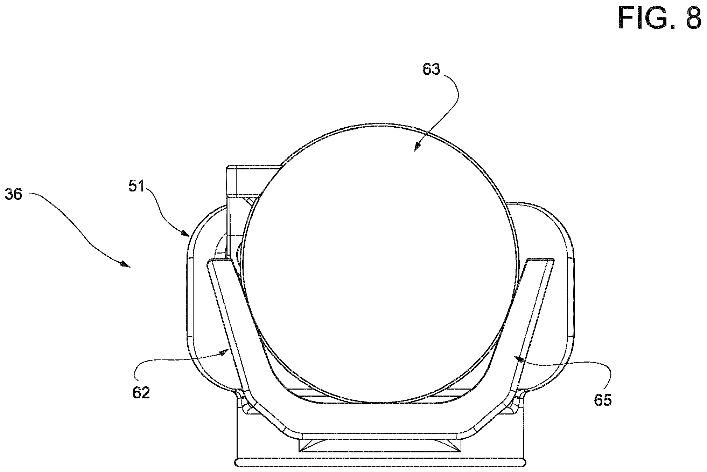

With particular reference to Figures 1 to 9, quality control apparatus 9 comprises a robot 19 arranged within inner environment 4. In particular, robot 19 is configured to operate in one or more operating configurations (the operating configurations being distinct from one another) so as to collect samples within inner environment 4 for determining the cleanliness and/or sterility within inner environment 4.

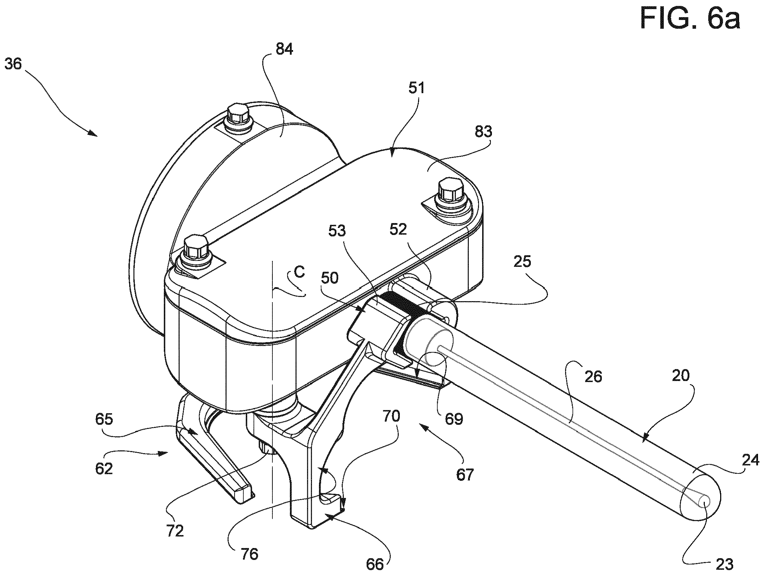

With particular reference to Figures 6a to 9, robot 19 is designed to manipulate and/or operate swab devices 20 and/or fluid filtering and/or sampling devices 21 and/or sampling dishes 22 (such as Petri dishes), in particular for collecting samples designed to determine information about possible contaminations (e.g. due to the presence of microbes, bacteria, viruses or any other contaminations) within inner environment 4.

In more detail and with particular reference to Figure 6a, each swab device 20 may allow to take a sample by swapping over a surface arranged within inner environment 4.

In even more detail, each swab device 20 may comprise a probe head 23, e.g. comprising cotton or similar, designed to be put into contact with a surface and to be guided over the surface for possibly collecting material present on the surface.

Preferentially, each swab device 20 may also comprise a tubular receptacle 24 designed to host and protect the respective probe head 23, in particular prior and after swabbing of the respective probe head 23.

Even more preferentially, each swab device 20 may also comprise a gripping portion 25 and a rod portion 26 carrying the respective probe head 23 at a first end and the respective gripping portion 26 at a second end.

According to some possible embodiments, each swab device 20 may comprise a securing assembly (e.g. a screwing mechanism) for (removably and/or reversibly) securing the respective gripping portion 25 to the respective tubular receptacle 24.

According to some possible non-limiting embodiments, swab devices 20 may be based on the detection of adenosine triphosphate (ATP).

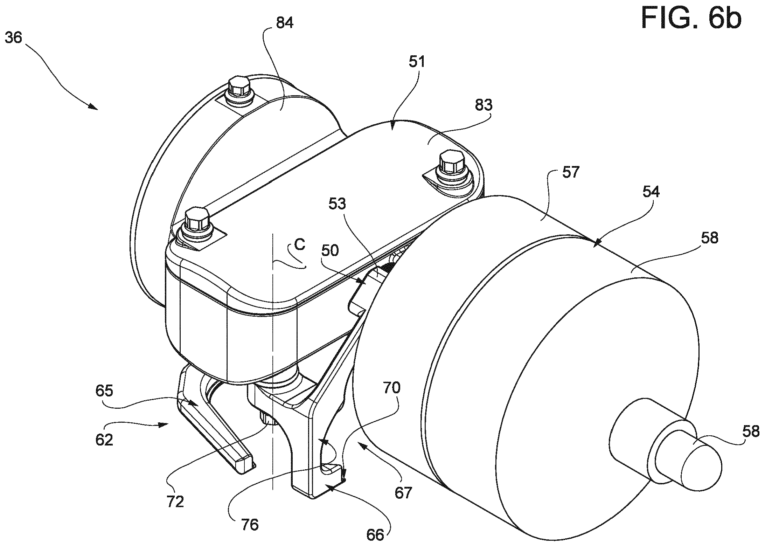

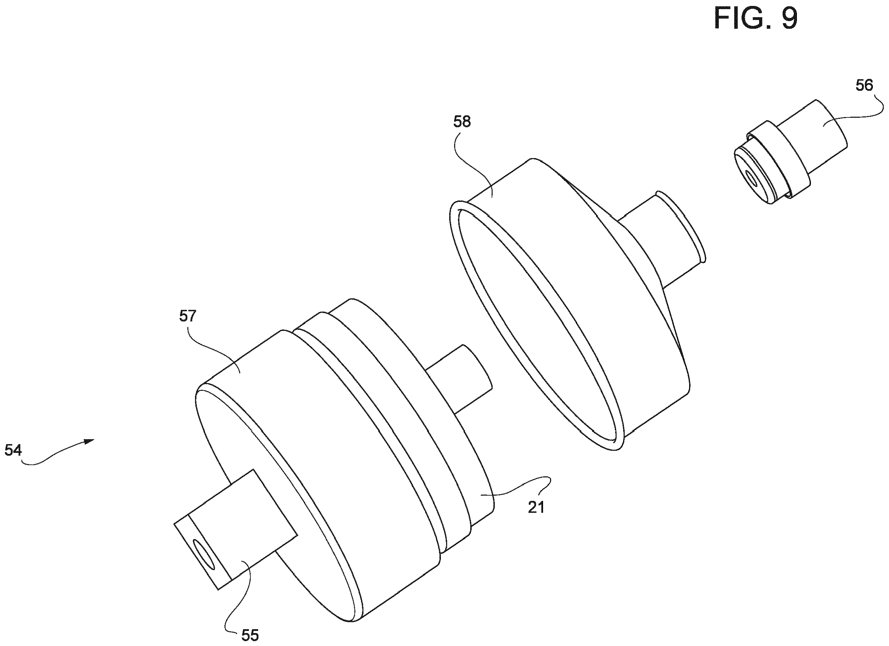

In more detail and with particular reference to Figures 6b and 9, each fluid filtering and/or sampling device 21 is designed to be fluidically coupled to a fluidic line within inner environment 4 so as to filter and/or sample the fluid being present within the fluidic line. In particular, such fluidic line may provide for a liquid (e.g. sterile water) or a gas (e.g. sterile air) to be used during operation of receptacle treatment machine 1.

Preferentially, each fluid filtering and/or sampling device 21 may be designed to at least retain contaminations possibly present within the filtered and/or sampled fluid.

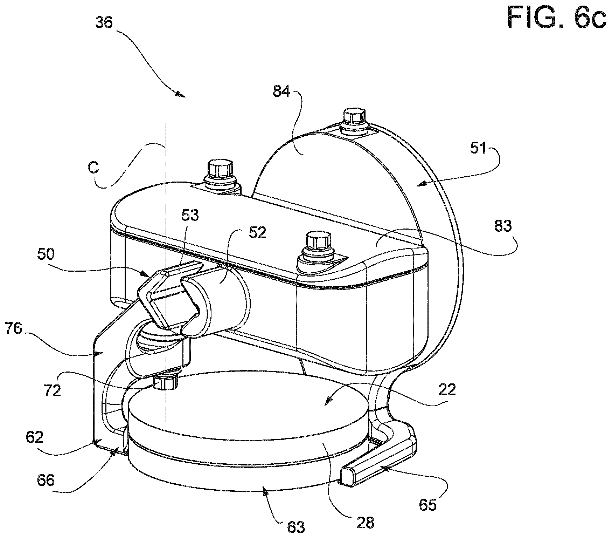

In more detail and with particular reference to Figures 6c to 8, each sampling dish 22 comprises a main dish 27 and a cover 28 (removably) placed and/or placeable onto main dish 27.

Furthermore, at least each sample dish 22, in particular the respective main dish 27, may contain a culture media.

With particular reference to Figures 1 and 2, robot 19 may be placed within inner environment 4 adjacent to filling apparatus 7 and/or capping apparatus 8, in particular so that the sampling can be executed at and/or adjacent to filling apparatus 7 and/or capping apparatus 8.

Moreover, robot 19 could be fixedly arranged (i.e. its position within inner environment 4 is not modifiable) or is moveable so as to modify, in use, the position of robot 19 within inner environment 4.

In more detail, robot 19 could comprise and/or could be coupled to an actuating unit for modifying its position within isolation chamber 3 and/or inner environment 4.

According to some possible non-limiting embodiments, the actuating unit could comprise a rail system and/or could be based on magnetic elevation and/or wheels coupled to robot 19 and/or defining a tracked robot 19.

Advantageously and with particular reference to Figures 1 to 9, robot 19 comprises at least a manipulating device 34 having a robotic arm 35 and an end effector unit 36 connected to the robotic arm 35.

Moreover, robot 19 and/or manipulating device 34 could comprise a control unit configured to control operation of robotic arm 35 and/or end effector unit 36.

Additionally, robot 19 could comprise a base carrying robotic arm 35.

In more detail, robotic arm 35 may be configured to control movement of end effector unit 36 in a three-dimensional space and/or may actuate a rotation of end effector unit 36 around a rotation axis B.

Preferentially, robotic arm 35 may be of the anthropomorphic-type.

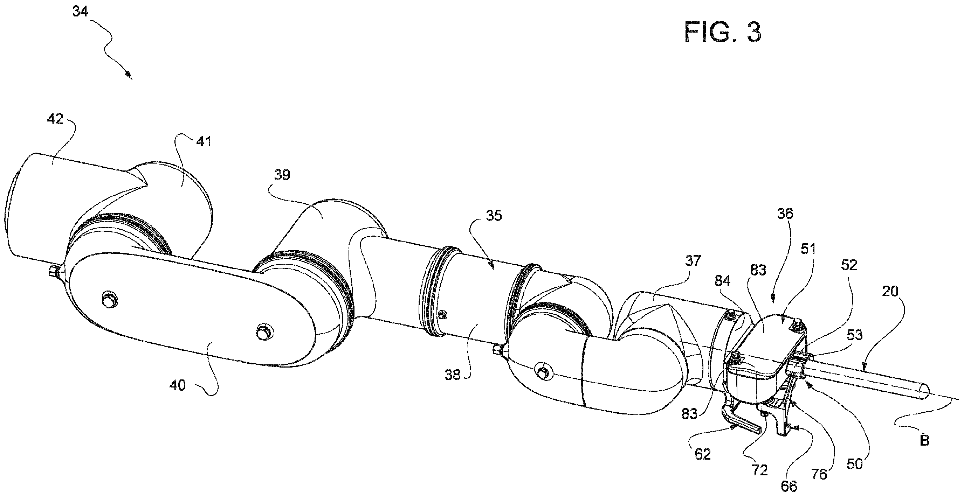

With particular reference to Figure 3, robotic arm 35 may comprise at least a wrist portion 37 carrying end effector unit 36.

Preferentially, end effector unit 36 may be rotatably mounted to wrist portion 37 allowing for rotation around rotation axis B. In particular, robotic arm 35 comprises an electrical motor arranged within wrist portion 37, being operatively coupled to end effector unit 36 and configured to actuate rotation of end effector unit 36 around rotation axis B.

Moreover, robotic arm 35 may comprise:
- a forearm portion 38 connected to wrist portion 37;
- an elbow portion 39 connected to forearm portion 38;
- a biceps portion 40 connected to elbow portion 39;
- a shoulder portion 41 connected to biceps portion 40; and
- a tower portion 42 connected to shoulder portion 40.

Preferentially, forearm portion 38 and wrist portion 37 and/or forearm portion 38 and elbow portion 39 and/or elbow portion 39 and biceps portion 40 and/or biceps portion 40 and should portion 41 and/or shoulder portion 41 and tower portion 42 are moveable, in particular angularly moveable, with respect to one another.

Preferentially, robotic arm 35 comprises a plurality of electrical motors designed to actuate the relative (angular) movements between forearm portion 38 and wrist portion 37 and/or forearm portion 38 and elbow portion 39 and/or elbow portion 39 and biceps portion 40 and/or biceps portion 40 and should portion 41 and/or shoulder portion 41 and tower portion 42.

Advantageously, robot 19, in particular manipulating device 34, even more particular end effector unit 36, is controllable into at least one, preferentially in all, of the following operating conditions:
- a first operating condition (see Figure 6a) within which robot 19, in particular end effector unit 36, is configured to manipulate at least one swab device 20 at a time;
- a second operating condition (see Figure 6b) within which robot 19, in particular end effector unit 36, is configured to manipulate at least one fluid filtering and/or sampling device 21 at a time; and
- a third operating condition (see Figure 6c) within which robot 19, in particular end effector unit 36, is configured to manipulate at least one sampling dish 22 at a time. The third operating condition can be considered as a further operating condition with respect to the first operating condition and/or with respect to the second operating condition.

Preferentially, robot 19, in particular manipulating device 34, even more particular end effector unit 36, can be repeatedly controlled in the first and/or second and/or third operating condition.

Preferentially, robot 19, in particular manipulating device 34, even more particular end effector unit 36, can be repeatedly (and in varying orders) controlled between the first, second and third operating condition.

Moreover, the control unit may be configured to control operation of robotic arm 35 and/or end effector unit 36 in dependence of the specific operating condition within which robot 19, in particular manipulating device 34, even more particular end effector unit 36, is controlled.

In more detail, the control unit is configured to control robotic arm 35 and/or end effector unit 36 such:
- to take at least one sample by manipulating at least one swap device 20 with manipulating device 34, in particular end effector unit 36, being controlled in the first operating condition; and/or
- to take at least one sample by manipulating at least one fluid filtering and/or sampling device 21 with manipulation device 34, in particular end effector unit 36, being controlled in the second operating condition; and/or
- to take at least one sample by manipulating at least one sample dish 22 with manipulation device 34, in particular end effector unit 36, being controlled in the third operating condition.

According to some possible non-limiting embodiments, receptacle treatment machine 1 may comprise a lock housing 43 having and/or delimiting a lock space 44. In particular, lock housing 43 is designed to allow a discharging of (used) swap devices 20 and/or (used) fluid filter and/or sampling devices 21 and/or (used) sampling dishes 22 from isolation chamber 3 during operation of receptacle treatment machine 1, in particular without the need to interrupt operation of receptacle treatment machine 1 and to maintain the cleanliness and/or sterility of inner environment 4.

In more detail, lock housing 43 comprises a first opening and a second opening allowing a fluidic connection of lock space 44 with respectively inner environment 4 and outer environment 5.

Preferentially, lock housing 43 also comprises means associated to the first and/or second opening so as to guarantee that during operation of receptacle treatment machine 1 contaminations may not enter from outer environment 5 into inner environment 4. Such means may comprise closing elements and/or gas (air) blades and/or directed gas (air) flows.

In further detail, the control unit may be configured to control robotic arm 35 and/or end effector unit 36 such that the manipulation of the at least one swap device 20 comprises one or more of the following (consecutive) steps:
- gripping swap device 20;
- engaging the respective tubular receptacle 24 on a retaining device (not shown) of receptacle treatment machine 1 for locking the respective tubular receptacle 24;
- extracting the respective probe head 23 out of the respective (and retained) tubular receptacle 24;
- moving a portion of swap device 20, in particular the respective probe head 23, over a sample surface (within inner environment 4), in particular the sample surface could be associated to isolation chamber 3 and/or conveying apparatus 6 and/or filling apparatus 7 and/or capping apparatus 8;
- placing the respective probe head 23 back into the respective tubular receptacle 24; and
- transferring the respective swab device 20 into lock housing 43 and/or lock space 44.

In further detail, the control unit may be configured to control robotic arm 35 and/or end effector unit 36 such that the manipulation of the at least one fluid filtering and/or sampling device 21 comprises one or more of the following (consecutive) steps:
- gripping the at least one fluid filtering and/or sampling device 21;
- fluidically connecting the at least one fluid filtering and/or sampling device 21 with a fluid line (transporting a fluid such as a (sterile) liquid or a (sterile) gas) of receptacle treatment machine 1;
- detaching the filtering and/or sampling device 21 from the fluid line; and
- transferring the respective filtering and/or sampling device 21 into lock housing 43 and/or lock space 44.

In further detail, the control unit may be configured to control robotic arm 35 and/or end effector unit 36 such that the manipulation of the at least one sampling dish 22 comprises one or more of the following (consecutive) steps:
- engaging sampling dish 22;
- carrying sampling dish 22;
- placing sampling dish 22 onto a support surface (e.g. of the base and/or being associated to isolation chamber 3 and/or conveying apparatus 6 and/or filling apparatus 7 and/or capping apparatus 8) within inner environment 4;
- releasing sampling dish 22;
- removing the respective cover 28 from the respective main dish 27;
- placing the respective cover 28 back on the respective main dish 27;
- newly engaging sampling dish 22;
- newly carrying sampling dish 22; and
- transferring sampling dish 22 into lock housing 43 and/or lock space 44.

According to some alternative embodiments, instead or as an alternative of transferring swap device 20 or fluid filtering and/or sampling device 21 or sampling dish 22, robotic arm 35 and/or end effector unit 36 may, in use, be controlled such to retain swap device 20 or fluid filtering and/or sampling device 21 and/or sampling dish 22 within inner environment 4. In particular, swap device 20 or fluid filtering and/or sampling device 21 or sampling dish 22 is/are discharged from inner environment 4 only after stopping operation of receptacles treatment machine 1.

With particular reference to Figures 3 to 5, end effector unit 36 may comprise at least a gripping assembly 50 configured to selectively (and directly or indirectly) grip and/or carry swab device 20 and/or fluid filtering and/or sampling device 21.

Preferentially, end effector unit 36 comprises a support structure 51, in particular rotatably mounted to wrist portion 37.

In particular, support structure 51 carries at least gripping assembly 50.

In more detail, gripping assembly 50 comprises at least a first clamp element 52 and a second clamp element 53, which are configured to (directly or indirectly) grip in collaboration with one another swab device 20 and/or fluid filtering and/or sampling device 21.

According to the embodiment shown, first clamp element 52 is (non-moveably and/or integrally) fixed to support structure 51 and second clamp element 53 is moveable with respect to first clamp element 52, in particular second clamp element 53 is moveably coupled to support structure 51.

In particular, second clamp element 53 is moveable between at least an active position and a rest position at which first clamp element 52 and second clamp element 53 are adapted to respectively execute a clamping step and a releasing or receiving step.

E.g. gripping assembly 50 is, in particular clamp elements 52 and 53 are, designed to engage and clamp gripping portions 25 for gripping the respective swab devices 20. In particular, clamp elements 52 and 53 are adapted to clamp gripping portion 25 with second clamp element 53 being in the active position and to release or receive gripping portion 25 with second clamp element 53 being in the rest position.

Furthermore and with particular reference to Figures 6b and 9, robot 19, in particular the manipulating device 34, may comprise a support housing 54 having an inner space housing or designed to house one respective fluid filtering and/or sampling device 21. In particular, support housing 54 allows to facilitate the gripping and handling of fluid filtering and/or sampling device 21 by gripping assembly 50, in particular by clamp elements 52 and 53.

In more detail, support housing 54 may comprise a gripping portion 55 designed to be clamped in between first and second clamp elements 52 and 53 such that gripping assembly 50 carries support housing 54 together with fluid filtering and/or sampling device 21.

In particular, support housing 43 is designed to allow gripping assembly 50 to indirectly carry fluid filter and/or sampling device 21.

In even further detail, support housing 43 may also comprise a coupling portion 56 designed to couple to one or more fluidic lines of receptacle treatment machine 1 and to establish the fluidic connection between fluid filtering and/or sampling device 21 and the fluidic line.

Additionally, support housing 43 may comprise a first housing portion 57 and a second housing portion 58 being removably connected or connectable with one another and delimiting the inner space of support housing 43. In particular, housing portion 57 carries gripping portion 55 and housing portion 58 carries coupling portion 56.

E.g. gripping assembly 50 is, in particular clamp elements 52 and 53 are, also designed to engage and clamp gripping portion 55 for gripping the respective support housing 54. In particular, clamp elements 52 and 53 are adapted to clamp gripping portion 55 with second clamp element 53 being in the active position and to release or receive gripping portion 55 with second clamp element 53 being in the rest position.

With particular reference to Figures 3 to 8, end effector unit 36 comprises a support device 62 configured to at least indirectly (in other words, directly or indirectly) carry one sampling dish 22.

Preferentially, robot 19, in particular manipulating device 34, may comprise a support platform 63 designed to support and carry sampling dish 22. In particular, support platform 63 comprises a cavity for receiving and hosting a portion of sampling dish 22, in particular main dish 27, and in particular a gasket 64, in the example shown an O-ring, for fixing sampling dish 22 within the cavity.

In particular, support device 62 is designed to carry support platform 63 for indirectly carrying sampling dish 62.

In more detail, support platform 63 is designed such to leave an interspace 73 between support platform 63 and cover 28. As will be apparent from further below such interspace 73 may facilitate removing or placing cover 28 from or to main dish 27.

Preferentially, support platform 63 comprises (or even consists) of a ferromagnetic or magnetic or paramagnetic material.

According to some preferred non-limiting embodiments, receptacle treatment machine 1 may comprise one or more magnets positioned so as to define placing stations at which, in use, support platform 63 should be placed together with the respective sampling dish 22. In particular, the magnets are designed to interact with support platform 63 so as to exert a retaining force on support platform 63. In particular, the magnets may be placed within outer environment 5 (and support platform 63 and the respective magnet are separated by means of a relative portion of walls 15).

In more detail, support device 62 comprises a fork element 65 (having two spaced apart arms), in particular fixedly (non-moveably and/or integrally) mounted to support structure 51 and an auxiliary support element 66 moveable with respect to fork element 65 (and support structure 51) between an operative position at which auxiliary support element 66 is configured to support in collaboration with fork element 65 sampling dish 22, e.g. by supporting support platform 63, and a release position at which auxiliary support element 66 is designed to be retracted from sampling dish 22, e.g. by retracting from support platform 63 (so as to allow receiving or releasing sampling disk 22 and/or support platform 63).

In more detail, fork element 65 delimits a cavity 67 designed to receive at least partially sampling disk 22 and/or support platform 63.

According to some preferred non-limiting embodiments, support device 62 comprises an engagement surface 68 configured to carry sampling disk 22 and/or support platform 63. In particular, fork element 65 and auxiliary support element 66 comprise respectively a first surface portion 69 and a second surface portion 70 of engagement surface 68.

In more detail, engagement surface 68 defines, in particular first surface portion 69 and second surface portion 70 define, at least three rest points for carrying sampling disk 22 and/or support platform 63 and with auxiliary support element 66 being in the operative position.

Preferentially, engagement surface 68 protrudes into and/or towards cavity 67 at least with auxiliary support element 66 being in the operative position.

According to the specific example shown, support platform 63 comprises an undercut 71 for interacting with engagement surface 68.

Advantageously, support device 62, in particular fork element 65 and auxiliary support element 66, even more particular engagement surface 68, may also be configured to engage with and to carry cover 28 so as to allow for removing and placing of cover from and to main dish 27.

In particular, interspace 73 allows to receive engagement surface 68 thereby facilitating the process of removing and placing cover 28 from and to main dish 27.

With particular reference to Figures 3, 5 and 6a to 6c, end effector unit 36 may also comprise a clamping element 72 configured to engage cover 28 and to collaborate with engagement surface 68 so as to exert a clamping fore, in particular thereby clamping support platform 63 and/or sampling dish 22 between clamping element 72 and engagement surface 68.

With particular reference to Figures 3 to 6c, end effector unit 36 may comprise:
- a support arm 76 angularly moveable about a rotation axis C and carrying (at one end) or defining second clamp element 53 and (at the other end) auxiliary support element 66; and
- an actuation device 77 configured to actuate an angular movement of support arm 76 around rotation axis C.

In particular, the angular movement of support arm 76 around rotation axis C allows controlling second clamp element 53 and auxiliary support element 66 between respectively the active and the rest position and the operative position and the release position.

In more detail, support arm 76, clamp element 53 and auxiliary support element 66 are realized in a single piece.

In more detail, actuation device 77 comprises a first shaft 78 (fixedly) connected with support arm 76 by means of a connection element 79 (of end effector unit 36) and defining rotation axis C. In particular, shaft 78 extends along and is rotatable around rotation axis C.

Preferentially, rotation axis C and support arm 76 may be substantially parallel to one another.

Preferentially, rotation axis C may be transversally, in particular perpendicularly, oriented with respect to fork element 65.

In even further detail, connection element 79 is transversally arranged both to shaft 78 and to support arm 76.

Preferentially, connection element 79 may be integral to support arm 76.

Moreover, clamping element 72 is connected to (a terminal portion of) shaft 78.

Additionally, clamping element 72 is designed to at least partially connect connection element 79 and thereby support arm 76 to shaft 78.

Moreover, actuation device 77 may comprise an electrical motor 80 having a drive axle 81 rotatable around a rotation axis E being not parallel and/or being transversal, in particular perpendicular, to rotation axis C, and being operatively coupled to shaft 78 such that rotation of drive axle 81 around rotation axis E actuates a rotation of shaft 78 around rotation axis C.

Additionally, actuation device 77 may comprise a gear assembly 82 operatively coupling shaft 78 and drive axle 81 with one another.

According to some preferred non-limiting embodiments, support structure 51 comprises a housing 83 within which electrical motor 80, and in particular gear assembly 82 and a portion of shaft 78, is/are placed.

Preferentially, housing 83 fixedly carries clamping element 52.

The support structure 51 defines or comprises the housing 83. The first clamp element 52 is constrained to the housing 83. The first clamp element 52 is constrained or fixed to the housing 83. The first clamp element 52 is located in front of the housing 83 along a direction which is not parallel to the rotation axis C of the support arm 76 and which is not parallel to the rotation axis E of the motor 80. Said direction not parallel to rotation axis C of support arm 76 is a direction transversal with respect to both the rotation axis C of the support arm 76 and the rotation axis E of the motor 80. The fork element 65 is located below the housing 83 along a direction which is parallel to the rotation axis C of the support arm 76 and which is not parallel to the rotation axis E of the motor 80. With this configuration, together with the configuration of the support arm 76 defining the second clamp element 53 and the auxiliary support element 66, it is improved the compactness of the end effector unit 36 which end effector unit 36 is controllable in the first operating condition and/or in the second operating condition, and which end effector unit 36 is controllable in the third operating condition.

Additionally, support structure 51 may comprise a coupling structure 84 rotatably (around rotation axis B) connected to wrist portion 37 and carrying housing 83 and fork element 65.

In use, receptacle treatment apparatus 1 fills receptacles 2 with the pourable product and/or applies and fastens closures on receptacles 2.

In more detail, conveying apparatus 6 advances receptacles 2 along advancement path P, filling apparatus 7 fills receptacles 2 with the pourable product during their advancement along filling portion P1 and capping apparatus 8 applies and fastens the closures on receptacles 2 during their advancement along capping portion P2.

In particular, advancement, filling and capping of receptacles 2 occur within inner environment 4, thereby inner environment 4 being a clean and/or sterile and/or aseptic environment.

During operation of receptacle treatment apparatus 1 also the cleanliness and/or sterility within inner environment 4 is controlled by relying on quality control apparatus 9, in particular robot 19.

In more detail, robot 19, in particular manipulating device 34, even more particular end effector unit 36, is (selectively) controlled into at least one of the first, second and third operating condition. Preferentially, during operation of receptacle treatment apparatus 1, robot 19, in particular end effector unit 36, is controlled at least once into the first operating condition, at least once into the second operating condition and at least once into the third operating condition.

Even more preferentially, during operation of receptacle treatment apparatus 1, robot 19, in particular manipulating device 34, even more particular end effector unit 36, is repeatedly controlled into the first, second and third operating condition so as to obtain samples at different times during the operation of receptacle treatment apparatus 1.

In further detail, robot 19, in particular manipulating device 34, even more particular end effector unit 36, is configured to:
- manipulate at least one swab device 20 at a time when being controlled in the first operating condition; and/or
- to manipulate at least one fluid filtering and/or sampling device 21 at a time when being controlled in the second operating condition; and/or
- to manipulate at least one sampling dish 22 at a time when being controlled in the third operating condition.

Moreover, the control unit may control operation of robotic arm 35 and/or end effector unit 36 in dependence of the specific operating condition within which robot 19, in particular end effector unit 36, is controlled.

Preferentially, one or more (new) swab devices 20, fluid filtering devices 21 and sampling dishes 22 are placed within inner environment 4, in particular prior to operation of receptacle treatment apparatus 1.

In more detail, robotic arm 35 and/or end effector unit 36:
- take at least one sample operating at least one swap device 20 with robot 19, in particular end effector unit 36, being controlled in the first operating condition; and/or
- take at least one sample operating at least one fluid filtering and/or sampling device 21 with robot 19, in particular end effector unit 36, being controlled in the second operating condition; and/or
- take at least one sample operating at least one sample dish 22 with robot 19, in particular end effector unit 36, being controlled in the third operating condition.

In even further detail, robotic arm 35 and/or end effector unit 36 are with robot 19, in particular end effector unit 36, controlled such that the manipulation of the at least one swap device 20 comprises one or more of the following (consecutive) steps:
- gripping swap device 20;
- engaging the respective tubular receptacle 24 on a retaining device (not shown) of receptacle treatment machine 1 for locking the respective tubular receptacle 24;
- extracting the respective probe head 23 out of the respective (and retained) tubular receptacle 24;
- moving a portion of swap device 20, in particular the respective probe head 23, over a sample surface (within inner environment 3);
- placing the respective probe head 23 back into the respective tubular receptacle 24; and
- transferring the respective swab device 20 into lock housing 43 and/or lock space 44.

According to some alternative embodiments, instead of the step of transferring, a step of retaining swab device 20 within inner environment 4 could be executed.

In particular, during the step of gripping swap device 20, gripping assembly 50 grips gripping portion 25. Even more particular, clamping element 53 is moved (by rotation of support arm 76 around rotation axis C, from the rest position to the active position such that clamping elements 52 and 53 clamp gripping portion 25 between one another. Preferentially, the rotation of support arm 76 is activated by actuation device 77.

In particular, during the steps of engaging and/or extracting and/or moving and/or placing robotic arm 35 is moved, while gripping assembly 50 continues retaining gripping portion 25. In some cases, it may also be necessary to actuate a rotation of end effector unit 36 around rotation axis B during the one or more of the steps of engaging and/or extracting and/or moving and/or placing.

In further detail, the control unit may control robotic arm 35 and/or end effector unit 36 such that the manipulation of the at least one fluid filtering and/or sampling device 21 comprises one or more of the following (consecutive) steps:
- gripping the at least one fluid filtering and/or sampling device 21;
- fluidically connecting the at least one fluid filtering and/or sampling device 21 with a fluid line (transporting a fluid such as a liquid or a gas) of receptacle treatment machine 1;
- detaching the filtering and/or sampling device 21 from the fluid line; and
- transferring the respective filtering and/or sampling device 21 into lock housing 43 and/or lock space 44.

According to some alternative embodiments, instead of the step of transferring, a step of retaining filtering and/or sampling device 21 within inner environment 4 could be executed.

In particular, during the step of gripping, gripping assembly 50 clamps gripping portion 55 so as to retain support housing 54 containing fluid filtering and/sampling device 21. Even more preferentially, clamping element 53 is moved (by rotation of support arm 76 around rotation axis C, from the rest position to the active position such that clamping elements 52 and 53 clamp gripping portion 55 between one another. Preferentially, the rotation of support arm 76 is activated by actuation device 77.

In further detail, the control unit may control robotic arm 35 and/or end effector unit 36 such that the manipulation of the at least one sampling dish 22 comprises one or more of the following (consecutive) steps:
- engaging sampling dish 22
- carrying sampling dish 22;
- placing sampling dish 22 onto a support surface (e.g. of the base) within inner environment 4;
- releasing sampling dish 22;
- removing the respective cover 28 from the respective main dish 27;
- placing the respective cover 28 on the respective main dish 27;
- newly engaging sampling dish 22;
- newly carrying sampling dish 22; and
- transferring sampling dish 22 into lock housing 43 and/or lock space 44.

According to some alternative embodiments, instead of the step of transferring, a step of retaining sampling dish 22 within inner environment 4 could be executed.

In particular, during the step of engaging or newly engaging, end effector unit 36 approaches sampling device 22 and/or support platform 63, in particular with auxiliary support element 66 being in the releasing position. Furthermore, once first surface portion 69 contacts support platform 63, auxiliary support element 66 is moved to the operative position.

In particular, during the step of carrying or newly carrying sampling dish 22 and/or support platform 63 is/are carried by support device 62, in particular being maintained by engagement surface 68.

In particular, during the step of placing, robot arm 35 moves end effector unit 36 so as to place sampling dish 22 and/or support platform 63 onto the support surface, preferentially at a placing station at which sampling dish 22 and/or support platform 63 are fastened by means of a magnetic force (resulting from the interaction with the respective magnet).

In particular, during the step of releasing executed once sampling dish 22 and/or support platform 63 is placed on the support surface, support device 62 is detached from sampling dish 22 and/or support platform 63. Even more particular, auxiliary support element 66 is controlled into the releasing position followed by retracting support device 62.

In particular, during the step of removing, support device 62 engages cover 28 and then end effector unit 36 is moved by robotic arm 35 away from main dish 27. Even more particular, at first surface portion 69 is positioned within interspace 73 and then auxiliary support element 66 is moved into the operative position.

In particular, during the step of placing, support device 62 carries cover 28 and then end effector unit 36 is moved by robotic arm 35 so as to approach main dish 27. Even more particular, engagement surface 68 carries cover 28 and once cover 28 is placed on main dish 27 auxiliary support element 66 is moved into the release position; afterwards, surface portion 69 is removed out of interspace 73.

In particular, during the step of transferring the respective swap device 20 or fluid filtering and/or sampling device 21 or sampling dish 22, robotic arm 35 moves and/or end effector unit 36 rotates around rotation axis B. Furthermore, end effector unit 36 releases swap device 20 or fluid filtering and/or sampling device 21 or sampling dish 22 within lock space 44.

According to some non-limiting embodiments, it may also be necessary to modify the position of robot 19 within inner environment 4, in particular so as to place robot 19 adjacent to lock housing 43.

Preferentially, after the step of transferring, a step of collecting and a step of analyzing are executed. During the step of collecting, the swap device 20 and/or fluid filtering and/or sampling device 21 and/or sampling dish 22 is/are removed from lock housing 43 and during the step of analyzing the swap device 20 and/or fluid filtering and/or sampling device 21 and/or sampling dish 22 is/are analyzed so as to determine the cleanliness within inner environment 4. The advantages of robot 19 and/or receptacle treatment machine 1 and/or the method according to the present invention will be clear from the foregoing description.

In particular, robot 19 allows to analyze the cleanliness and/or sterility within isolation chamber 3 during operating of receptacle treatment machine 1 by manipulating swap device(s) 20 and/or fluid filtering and/or sampling device(s) 21 and/or sampling dish(es) 22. Even more particular, robot 19 can execute different test procedures.

Another advantage resides in that end effector unit 36 comes along with a compact design. End effector unit 36 has a single electrical motor 80, which can actuate a rotation of support arm 76, which allows to control both gripping assembly 50 and support device 62.

A further advantage resides in that receptacle treatment machine 1 has lock housing 43 allowing to discharge swap device(s) 20 and/or fluid filtering and/or sampling device(s) 21 and/or sampling dish(es) 22 out of inner environment 4 without compromising the cleanliness of inner environment 4.

Clearly, changes may be made to robot 19 and/or receptacle treatment machine 1 and/or the method as described herein without, however, departing from the scope of protection as defined in the accompanying claims.

## Claims

1. Manipulating device (34) for a robot (19) for operation within a receptacle treatment machine (1), even more particular for operation within an aseptic and/or sterile environment of a receptacle treatment machine;
wherein the manipulating device (34) comprises:
- a robotic arm (35); and
- an end effector unit (36) mounted to the robotic arm (35);
wherein the end effector unit (36) is controllable into at least:
- a first operating condition within which the end effector unit (36) manipulates a swab device (20); and/or
- a second operating condition within which the end effector unit (36) manipulates a fluid filtering and/or sampling device (21); and
wherein the end effector unit (36) is controllable in a third operating condition within which the end effector unit (36) manipulates a sampling dish (22).

2. Manipulating device according to claim 1, wherein the robotic arm (35) and/or the end effector unit (36) are designed such:
- to move a portion of the swap device (20) over a sample surface if the end effector unit (36) is controlled into the first operating condition; and/or
- to fluidically connect the fluid filtering and/or sampling device (21) within a fluid line of the receptacle treatment machine (1) if the end effector unit (36) is controlled into the second operating condition; and
wherein the robotic arm (35) and/or the end effector unit (36) are designed as to place a sampling dish (22) onto a support surface and to remove a cover (28) of the sampling dish (22) if the end effector unit (36) is controlled into the third operating condition.

3. Manipulating device according to claim 1 or 2, wherein the end effector unit (36) comprises at least a gripping assembly (50) for gripping the swab device (20) and/or the fluid filtering and/or sampling device (21).

4. Manipulating device according to claim 3, wherein the gripping assembly (50) comprises a first clamp element (52) fixed to a support structure (51) of the end effector unit (36) and a second clamp element (53) moveable between an active position and a rest position;
wherein the first clamp element (52) and the second clamp element (53) are adapted to execute a clamping step and a releasing or receiving step with the second clamp element (53) being controlled in respectively the active position and the rest position.

5. Manipulating device according to claim 4, further comprising a support housing (54) having an inner space for the fluid filtering and/or sampling device (21);
wherein the support housing (54) comprises a gripping portion (55);
wherein the first clamp element (52) and the second clamp (53) are configured to clamp the gripping portion (55) between one another for carrying the support housing (54) together with the fluid filtering and/or sampling device (21).

6. Manipulating device according to any one of the preceding claims, wherein the end effector unit (36) comprises a support device (62) configured to at least indirectly support a sampling dish (22);
wherein the support device (62) comprises a fork element (65) and an auxiliary support element (66) moveable with respect to the fork element (65) between an operative position at which the auxiliary support element (66) is configured to support in collaboration with the fork element (65) the sampling dish (22) and a release position at which the auxiliary support element (66) is designed to be retracted from the sampling dish (22).

7. Manipulating device according to claim 6, further comprising a support platform (63) having a cavity for receiving and housing the sampling dish (22);
wherein the support device (62) is configured to carry the support platform (63) for indirectly supporting the sampling dish (22).

8. Manipulating device according to claim 4 or 5 and to claim 6 or 7, comprising:
- a support arm (76) angularly moveable about a rotation axis (C) and defining the second clamp element (53) and the auxiliary support element (66); and
- an actuation device (77) configured to actuate an angular movement of the support arm (76) around the rotation axis (C); the actuating device (77) comprising a motor (80) having a drive axle (81) rotatable around an auxiliary rotation axis (E).

9. Manipulating device according to claim 8, wherein the auxiliary rotation axis (E) is transversal or not parallel to the rotation axis (C) of the support arm (76);
wherein the support structure (51) defines an housing (83) where the motor (80) is housed, the first clamp element (52) being mechanically constrained to the housing (83) and being located in front of the housing (83) along a direction which is not parallel to the rotation axis (C) of the support arm (76) and which is not parallel to the rotation axis (E) of the motor (80), the fork element (65) being located below the housing (83) along a direction which is parallel to the rotation axis (C) of the support arm (76) and which is not parallel to the rotation axis (E) of the motor (80).

10. Robot (19) for operation within a receptacle treatment machine (1), even more particular for operation within an aseptic and/or sterile environment of a receptacle treatment machine (1);
wherein the robot (19) comprises at least one manipulating device (34) according to any one of the preceding claims.

11. Robot according to claim 10, wherein the robotic arm is configured to move the end effector unit within a three-dimensional space and/or to rotate the end effector unit (36) around a rotation axis (B) ; in particular the robotic arm (35) is an anthropomorphic robot arm.

12. Robot according to claim 11, further comprising a control unit configured to control operation of the robotic arm (35) and the end effector unit (36) in dependence of the operating condition of the end effector unit (36).

13. Receptacle treatment machine (1) for at least filling of receptacles (2) with a pourable food product and/or apply closures onto receptacles (2) filled with a pourable food product comprising at least:
- an isolation chamber (3) separating an inner environment (4), in particular an inner sterile environment (4), from an outer environment (5);
- a conveying apparatus (6) configured to advance the receptacles (2) along an advancement path (P) within the inner environment (4);
- a filling apparatus (7) at least partially arranged within the inner environment (4) and configured to fill the receptacles (2) with the pourable product during their advancement along at least a filling portion (PI) of the advancement path (P); and/or
- a capping apparatus (8) at least partially arranged within the inner environment (4) and configured to apply closures onto the receptacles (2) during their advancement along at least a capping portion (P2) of the advancement path (P);
wherein the receptacle treatment machine (1) further comprises at least one robot (19) according to any one of claims 10 to 12.

14. Receptacle treatment machine according to claim 13, further comprising a lock housing (43);
wherein the robot (19) is configured to place during operation of the receptacle treatment machine (1) swab devices (20) and/or sampling dishes (22) and/or fluid filtering and/or sampling devices (21) within the lock housing (43).

15. Method of controlling the cleanliness within a sterile and/or aseptic environment of a receptacle treatment machine (1);
the method comprises at least the steps of:
- providing a robot (19) having at least a robotic arm (35) and an end effector unit (36) within the sterile and/or aseptic environment;
- placing at least one swab device (20) and/or at least one fluid filtering and/or sampling device (21) and/or at least one sampling dish (22) within the sterile and/or aseptic environment (4);
- controlling the end effector unit (36) at least into one operating condition; and
- executing at least one manipulation step chosen in dependence of the operating condition during which the robotic arm (35) and/or the end effector unit (36) manipulate the swap device (20) or the fluid filtering and/or sampling device (21) or the sampling dish (22).
